# EUROPEAN PATENT APPLICATION

(11) **EP 2 517 731 A1**
(43) Date of publication of application: **31.10.2012**
(21) Application number: 11161568.8
(22) Date of filing: 07.04.2011
(51) Int. Cl.: A61K 48/00, C07K 14/435, C12N 15/09

(54) **Method of activating a target gene in a cell**

(71) Applicant: Ludwig-Maximilians-Universität München, 80539 München (DE)
(72) Inventor: Lahaye, Thomas, 82131 München (DE); Leonhardt, Heinrich, 80469 München (DE); Morbitzer, Robert, 82061 Neuried (DE); Bultmann, Sebastian, 82140 Olching (DE)
(74) Representative: Isarpatent

(57) **Abstract**

The present invention is directed to a method of activating expression of a target gene in a cell involving the treatment with at least one transcription activator like effector (TALE) polypeptide and with at least one epigenetic inhibitor substance, and an isolated cell obtainable by said method. The present invention is further directed to the use of said substances for activating expression of a target gene in a cell as well as to a composition or kit-of-parts comprising the same.

<110>  Ludwig-Maximilians-Universität

<120>  METHOD OF ACTIVATING A TARGET GENE IN A CELL

<130>  P31310-EP

<160>  7

<170>  PatentIn version 3.3

<210>  1
<211>  39
<212>  DNA
<213>  artificial

<220>
<223>  forward primer

<400>  1
ggggcgatcg cacaagtttg tacaaaaaag ctgaacgag                              39


<210>  2
<211>  38
<212>  DNA
<213>  artificial

<220>
<223>  reverse primer

<400>  2
ggggcggccg caaccacttt gtacaagaaa gctgaacg                               38


<210>  3
<211>  44
<212>  DNA
<213>  artificial

<220>
<223>  forward primer

<400>  3
gggggtctct caccatggat cctgcccccc cgaccgatgt cagc                        44


<210>  4
<211>  41
<212>  DNA
<213>  artificial

<220>
<223>  reverse primer

<400>  4
gggggtctcc cttctaccca ccgtactcgt caattccaag g                           41


<210>  5
<211>  58
<212>  DNA
<213>  artificial

<220>
<223>  linker oligonucleotide

<400>  5
cctaggtgag ccgtctttcc accaggcccc cggctcgggg tgcgatcgcc gcccatgg         58

<210>  6
<211>  23
<212>  DNA
<213>  artificial

<220>
<223>  forward primer

<400>  6
aaaggcgcca gtgagcaagg gcg                                                23


<210>  7
<211>  30
<212>  DNA
<213>  artificial

<220>
<223>  reverse primer

<400>  7
aaaggccggc cttacttgta cagctcgtcc                                         30

## Description

### TECHNICAL FIELD OF THE INVENTION

The present invention is directed to a method of activating expression of a target gene in a cell involving the treatment with at least one transcription activator like effector (TALE) polypeptide and with at least one epigenetic inhibitor substance, and an isolated cell obtainable by said method. The present invention is further directed to the use of said substances for activating expression of a target gene in a cell as well as to a composition or kit-of-parts comprising the same.

### BACKGROUND OF THE INVENTION

Transcription factors tend to bind short and partially degenerated DNA sequences and have multiple binding sites in the genome limiting their applicability for site specific genome manipulations. Past approaches to generate recombinant transcription factors utilized predominantly tandem arranged zinc fingers to target user defined target sequences but required labor-intensive optimization cycles and were limited by the range of accessible DNA target sequences.

Promising new strategies take advantage of DNA binding modules¹⁻⁴ naturally used by plant pathogen *Xanthomonas* spp. to exert transcriptional control in host cells. These transcription activator like effectors (TALEs) consist of a central domain of tandem repeats, nuclear localization signals (NLS) and an acidic transcriptional activation domain⁵. The central repeat domain consists of almost identical tandem arranged 33-34 amino acid motifs that collectively mediate DNA binding^{6,7}. TALE repeats differ predominantly in position 12 and 13, the so-called repeat variable diresidues (RVDs) each mediating interaction with a single base in a matching target sequence. Systematic studies⁸ established that individual RVDs possess distinct base preferences providing a rational basis for the direct design of TALEs against any desired DNA target sequence³.

The production of the so called TALEs has been intensively described, see e.g. Boch, J. et al. "Breaking the code of DNA binding specificity of TAL-type III effectors". Science 326, December 11, 2009, p. 1509-1512 or Zhang, F. et al. "Efficient construction of sequence-specific TAL effectors for modulating mammalian transcription" Nat Biotech Vol. 29, no. 2, February 2011, p. 149-153. Zhang et al. describe that they synthesized 17 TALEs that are customized to recognize specific DNA-binding sites, and demonstrate that these can specifically modulate transcription of endogenous genes (*SOX2* and *KLF4*) in human cells.

Furthermore, the use of TALEs for the activation and/or regulation of selected gene-loci is known from Christian et al, "Targeting DNA double-dtrand breaks with TAL effector nucleases". Genetics 186, 757-761 (2010).

Morbitzer et al. "Regulation of selected genome loci using de novo-engineered transcription activator-like effector (TALE)-type transcription factors", 14, December 2010, Proc NatI Acad Sci USA Vol 107, no. 50, (2010) 107, 21617-21622 describe the use of TALEs for the regulation of gene *Bs3* and *Bs4c* in capsicum species (pepper).

Miller et al., "A TALE nuclease architecture for efficient genome editing", Nat. Biotech, Vol 29, no. 2, February 2011, p. 143 - 148 describe the activation of the endogenous human *NTF3*-gene and showed that designed TALEs can regulate endogenous mammalian genes. They further demonstrated the effective application of designed TALE transcription factors and nucleases for the targeted regulation and modification of endogenous genes.

WO 2010/079430 A1 is directed to modular DNA-binding domains and methods of use thereof. More precisely, this application provides a method for producing TALE polypeptides that selectively recognize a base pair in a DNA sequence. This document further provides a method for targeted modulation of gene expression by constructing modular repeat units specific for a target DNA sequence of interest by using such a polypeptide for modulating the expression of a target gene in a cell as a result of such a recognition.

WO 2009/134714 A2 describes fusion molecules of DNA-binding proteins and effector domains wherein the effector domain might be selected from a repression domain such as a HDAC-domain.

Herman et al. "Histone deacetylase inhibitors reverse gene silencing in Friedreich's Ataxia" Nat. Chem. Biol, vol. 2, no. 10, October 2006 describe the synthesis and characterisation of a class of histone deacetylase inhibitors that reverse *FXN* (the gene encoding frataxin) silencing in primary lymphocytes from individuals suffering from Friedreich's ataxia. They conclude that the class of HDAC inhibitors may yield therapeutics for Friedreich's ataxia.

The TALE technology was successfully used to specifically activate mammalian genes but the efficiency varied greatly among the targeted genomic loci^{1,2} and depending on the epigenetic state of the target gene. This uncertainty limits the potential fields of applications considerably and thus forms a major drawback of the existing TALE technology.

Therefore, there is a need to overcome the problems existing with the current TALE technology and to provide a method of activating expression of a target gene in a cell providing enhanced efficiency independent of the targeted genomic loci.

### SUMMARY OF THE INVENTION

The above mentioned object is achieved by the subject matter of the independent claims. Preferred embodiments are indicated in the depending claims.

The invention provides a method of activating expression of a target gene in a cell, wherein the cell is treated with at least one transcription activator like effector (TALE) polypeptide or a functional part thereof and with at least one epigenetic inhibitor substance.

By the approach used in the present invention, i.e. by a combined treatment of the cell with the above indicated substances, it was demonstrated that the efficiency of the transcriptional activation of silenced/methylated genes can be greatly enhanced when using the TALE technology. It was demonstrated by the inventors that the epigenetic state of the target gene, e.g. promoter, is a crucial aspect in TALE mediated gene activation and, thus, the invention was concluded.

By using the present approach, the existing TALE technology will be greatly improved, opening up the possibility to use it efficiently in several applications such as the generation of pluripotent stem cells starting from differentiated cells, for use in the treatment of several diseases, for example, neurodegenerative and psychiatric disorders or cancer as well as in different applications in agriculture, biotechnology and medicine. Further, the present approach may be used in cell based therapies and regenerative medicine.

The invention discloses the use of a composition of TALE polypeptides and epigenetic inhibitor substances for activating expression of a target gene in a cell. The invention furthermore provides cells, in particular pluripotent stem cells, which are obtainable by the disclosed method.

Not being wanted to be bound to a specific theory, the inventors assume that the drawbacks involved in the actually available TALE technology is due to the fact that silenced gene sequences are condensed by histone deacetylases and, therefore, cannot be easily accessed by TALE polypeptides for subsequent activation. The inventors learned that the addition of an epigenetic inhibitor substance may overcome this problem, therefore, enhancing the activation of expression of a target gene in a cell provided by TALE polypeptides dramatically.

### DESCRIPTION OF THE FIGURES

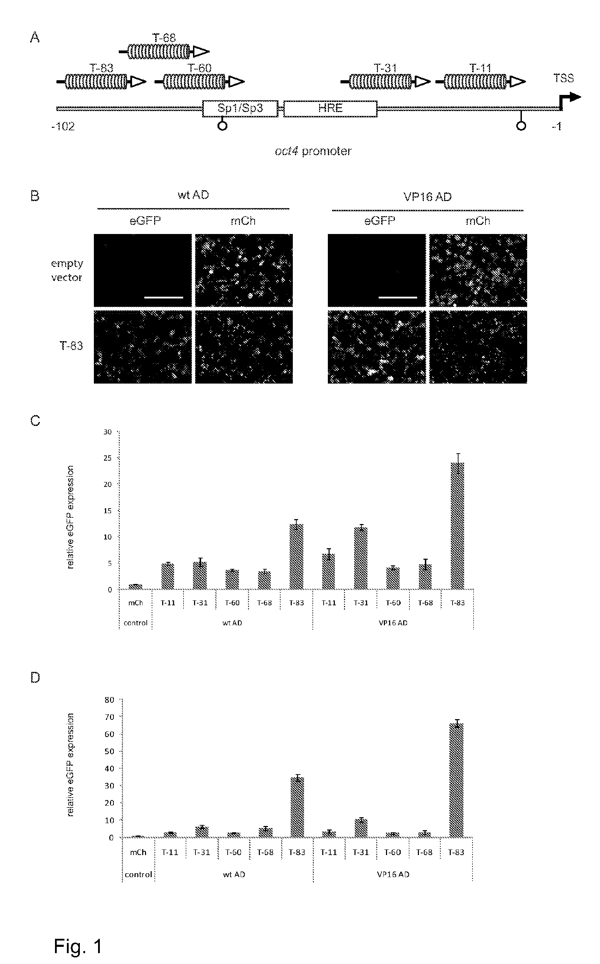

Figure 1: Activation of a transgenic *oct4* reporter construct by *dTALEs* in HEK293T cells (A) Schematic representation of the *oct4* proximal promoter showing the 102 base pairs (bp) upstream of the transcriptional start site (TSS) including the binding site of the Sp1/Sp3 transcription factors (Sp1/Sp3), the hormone responsive element (HRE) and two CpG sites (open circles). *oct4* dTALEs are depicted according to the location of their target sequence and numbered according to the distance between the 5' end of the targeted box and the TSS of the *oct4* endogen (B) Fluorescence microscopy images of HEK293T cells co-transfected with the p*oct4*-GFP reporter construct and the T-83 *dTALE* construct. Left panel shows cells transfected with the T-83 *dTALE* fused to the wildtype AD (wt AD). Right panel shows cells transfected with the T-83 *dTALE* fused to the VP16 AD. Scale bar represents 200 µm. (C) Transcriptional activation of the unmethylated p*oct4*-GFP reporter construct by *oct4 dTALEs*. eGFP expression was normalized to cells transfected with the methylated reporter and control plasmid (mCh). Error bars represent standard deviation from 3 independent experiments. (D) Transcriptional activation of the *in vitro* methylated p*oct4*-GFP reporter construct by *oct4 dTALEs*. eGFP expression was normalized to cells co-transfected with the methylated reporter and control plasmid (mCh). Error bars represent standard deviation from 3 independent experiments.

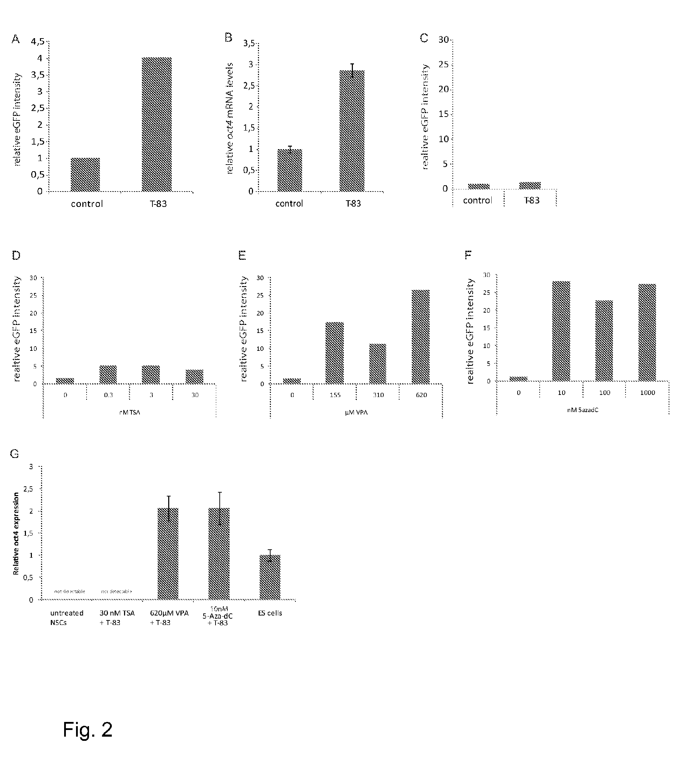

Figure 2: Activation of the endogenous *oct4* gene in ESCs and NSCs. (A) Relative eGFP intensities of mCherry positive ogESCs transfected with either control plasmid or T-83 *dTALE* construct (T-83) as measured by flow cytometry. (B) Relative *oct4* mRNA levels measured by quantitative real-time PCR of transfected, mCherry positive cells shown in (A). Error bars represent standard deviation of three technical replicates. (C) Relative eGFP intensities eGFP of mcherry positive ogNSCs. Cells transfected with either control plasmid or T-83 *dTALE* construct (T-83) are shown. (D) Relative eGFP intensities of transfected cells treated with different concentrations of TSA. eGFP intensities were measured by FACS, normalized to cells transfected with control plasmid and treated with the respective TSA concentration. (E) Relative eGFP intensities of transfected cells treated with different concentrations of VPA. eGFP intensities were measured by FACS, normalized to cells transfected with control plasmid and treated with the respective VPA concentration. (F) Relative eGFP intensities of transfected cells treated with different concentrations of 5azadC. eGFP intensities were measured by FACS, normalized to cells transfected with control plasmid and treated with the respective 5azadC concentration.

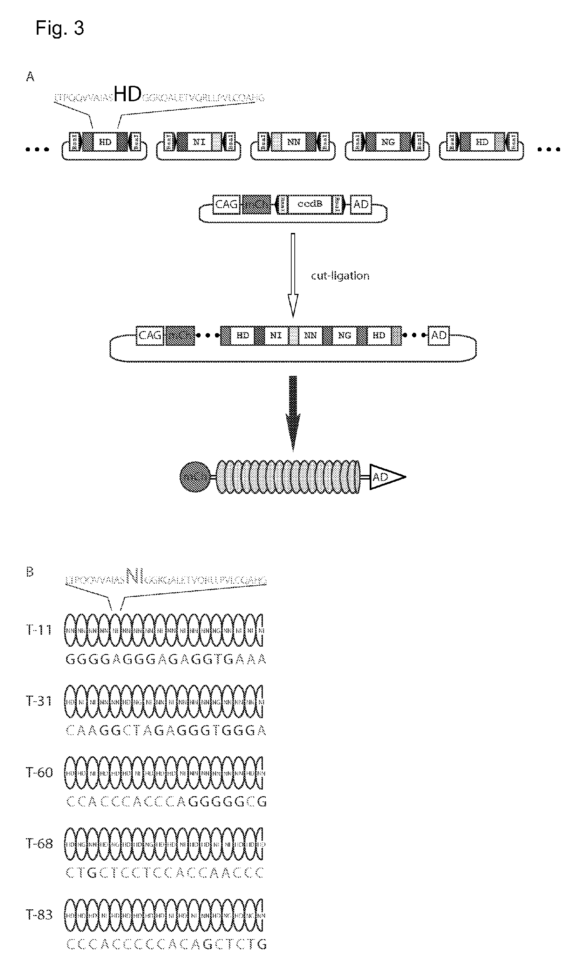

Figure 3: (A) Schematic representation of the strategy used to generate *dTALE* fusions. TALE repeat arrays were generated via multi-fragment cut-ligation using golden gate cloning. Subsequently, repeat arrays were ligated into the modified CMV early enhancer/chicken beta actin (CAG) promoter ―mCherry (mCh) vector. The expression cassette is followed by an internal ribosomal entry site (IRES) and a Blasticidin (Blast) resistance gene for selection in mammalian cells (B) Repeat variable diresidues (RVDs) of *dTALEs* and their recognized DNA sequences.

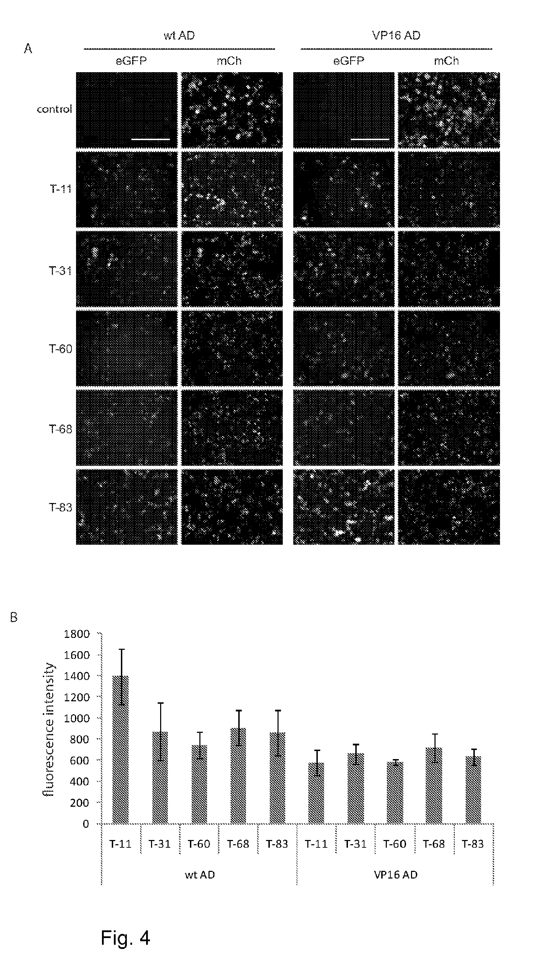

Figure 4: (A) Fluorescence microscopy images of HEK293T cells co-transfected with the p*oct4*-GFP reporter construct and the respective *dTALE* construct. Left panel shows cells transfected with the *dTALEs* fused to the wildtype AD (wt AD). Right panel shows cells transfected with *dTALEs* fused to the VP16 AD. Scale bar represents 200 µm. (B) Fluorescence intensity of mCherry in HEK293T cells transfected with the *oct4 dTALE* constructs from (A). Error bars represent standard deviation of three independent experiments.

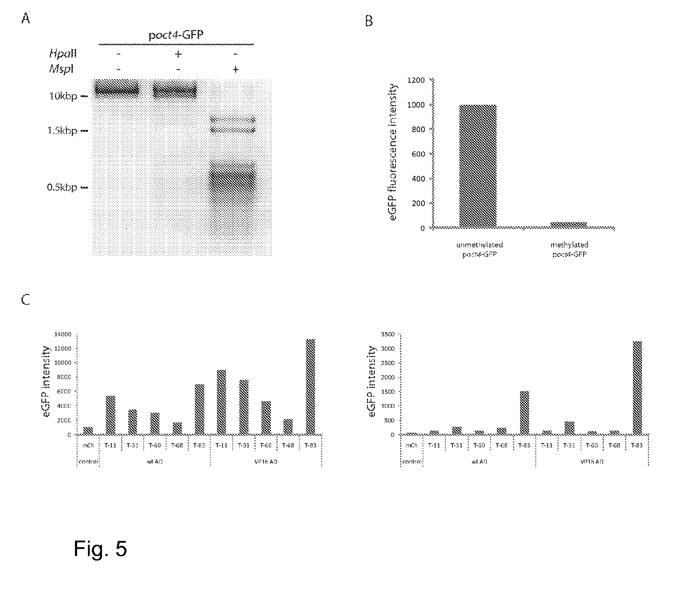

Figure 5: (A) Digestion of *in vitro* methylated p*oct4*-GFP with the methylation sensitive restriction enzyme Hpall and its methylation insensitive isoschizomere *Msp*I. (B) Absolute eGFP intensity of control cells transfected with unmethylated and methylated reporter plasmid. (C) Left panel: Absolute eGFP intensity of HEK293T cells co-transfected with the unmethylated p*oct4*-GFP construct and respective *dTALEs*. Right panel: Absolute eGFP intensity of HEK293T cells co-transfected with the *in vitro* methylated p*oct4*-GFP construct and respective *dTALEs.*

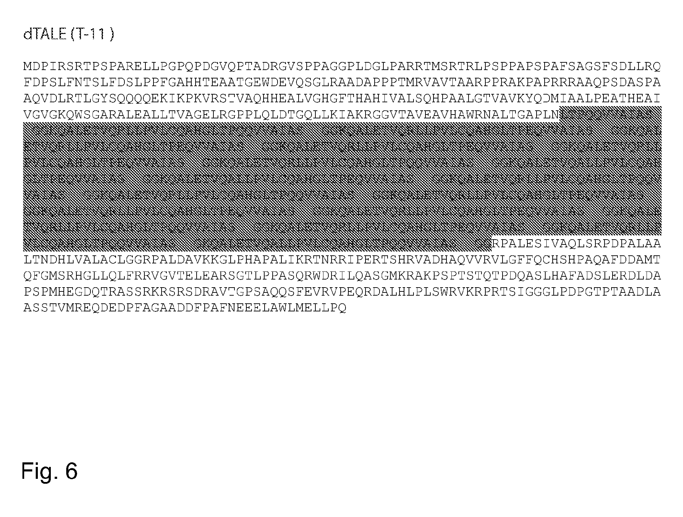

Figure 6: Amino acid sequences of *dTALE* T-11. Central repeat domain is highlighted in blue and RVDs in red. For RVDs of other *dTALE* constructs see figure 3B.

### DETAILED DESCRIPTION OF THE INVENTION

According to a first aspect, the present invention is directed to a method of activating expression of a target gene in a cell, wherein the cell is treated with at least one transcription activator like effector (TALE) polypeptide or a functional part thereof, characterized in that the cell is further treated with at least one epigenetic inhibitor substance.

The term "activating expression" of a target gene refers to the ability of a TALE polypeptide of the present invention to activate transcription of a gene. Activation includes prevention of subsequent transcriptional inhibition (i.e., prevention of repression of gene expression). This can be assayed by determining any parameter that is indirectly or directly affected by the expression of the target gene. Such parameters include, e.g., changes in RNA or protein levels, changes in protein activity, changes in product levels, changes in downstream gene expression, changes in reporter gene transcription (luciferase, CAT, beta-galactosidase, GFP (see, e.g., Mistili & Spector (1997) Nature Biotechnology 15:961-964); changes in signal transduction, phosphorylation and dephosphorylation, receptor-ligand interactions, second messenger concentrations (e.g., cGMP, cAMP, lP3, and Ca2+), cell growth, neovascularization, in vitro, in vivo, and ex vivo. Such functional effects can be measured by any means known to those skilled in the art, e.g., measurement of RNA or protein levels, measurement of RNA stability, identification of downstream or reporter gene expression, e.g., via chemiluminescence, fluorescence, calorimetric reactions, antibody binding, inducible markers, ligand binding assays; changes in intracellular second messengers such as cGMP and inositol triphosphate (lP3); changes in intracellular calcium levels; cytokine release, and the like.

"Activating expression" in particular means an enhancement of transcription or translation including a significant change in transcription or translation level of at least 1.5 fold, more preferably at least two fold, and even more preferably at least five fold.

Any target gene can be modulated by the present method. For example, the target gene can encode a product that affects biosynthesis, modification, cellular trafficking, metabolism and degradation of a peptide, a protein, an oligonucleotide, a nucleic acid, a vitamin, an oligosaccharide, a carbohydrate, a lipid, or a small molecule.

In one embodiment, a target gene comprises a transcriptional control element for a gene for which a desired phenotypic result can be attained by altering the degree of its expression. A transcriptional control element includes positive and negative control elements such as a promoter, an enhancer, other response elements, e.g., steroid response element, heat shock response element, metal response element, a repressor binding site, operator, and/or a silencer. The transcriptional control element can be viral, eukaryotic, or prokaryotic.

As such, the term "gene" as used herein generally refers to a nucleic acid molecule or portion thereof which comprises a coding sequence, optionally containing introns, and control regions which regulate the expression of the coding sequence and the transcription of untranslated portions of the transcript. Thus, the term "gene" includes, besides coding sequence, regulatory sequence such as the promoter, enhancer, 5' untranslated regions, 3' untranslated region, termination signals, polyadenylation region and the like. Regulatory sequence of a gene may be located proximal to, within, or distal to the coding region.

A preferred example of a promoter is a pluripotency gene promoter, preferably the oct4 pluripotency gene promoter.

In a preferred embodiment, the target gene comprises a transcriptionally or epigenetically silenced promoter or enhancer. The term "transcriptionally silenced" as used herein means those target genes, which have been silenced in a non-permanent manner. That is to say, this term describes a scenario where a specific target gene has been temporarily inactivated or silenced due to specific metabolic requirements etc.

In contrast thereto, the term "epigenetically silenced" as used herein shall mean a permanent inactivation of a target gene, for example in a fully differentiated cell.

It is noted that the status of gene silencing usually will be between both of these extreme positions (and thus will not fulfill one of these states by 100%).

Gene silencing is generally used to describe the "switching off" of a gene. Gene silencing is the result of histone modifications, creating an environment of heterochromatin around a gene that makes it inaccessible to the transcriptional machinery (RNA polymerase, transcription factors, etc.). The approach of the present invention exactly starts at this point which presumably is responsible for the variations in the efficiency of TALE polypeptides in gene activation.

The TALE polypeptides as used herein have been described in the prior art in detail. For example, it is referred to the above mentioned publication WO 2010079430, which is incorporated herein in its entirety by reference. In particular, it is referred to the definition of TALE polypeptides and the way how to identify/produce them.

Members of the TALE family are naturally translocated via the type III secretion system into plant cells. The type member of this effector family is AvrBs3. Hence, the TAL effector family is also named AvrBs3-like family of proteins. Both expressions are used synonymously and can be interchanged. Non-limiting examples of the AvrBs3-like family are as follows: AvrBs4 and the members of the Hax sub-family Hax2, Hax3, and Hax4 as well as Brg11. AvrBs3 and the other members of its family are characterized by their binding capability to specific DNA sequences in promoter regions of target genes and induction of expression of these genes. They have conserved structural features that enable them to act as transcriptional activators of plant genes. AvrBs3-like family and homologous effectors typically have in their C-terminal region nuclear localisation sequences (NLS) and a transcriptional activation domain (AD). The central region contains repeat units of typically 34 or 35 amino acids. The repeat units are nearly identical, but variable at certain positions and it has now been found how these positions determine the nucleotide sequence binding specificity of the proteins.

It was shown for AvrBs3 that the repeat units are responsible for binding to DNA. The DNA-binding specificity of AvrBs3 and probably other members of the AvrBs3-family seems to be mediated by the central repeat domain of the proteins. This repeat domain consists in AvrBs3 of 17.5 repeat units and in homologous proteins is comprised of 1.5 to 33.5 repeat units which are typically 34 amino acids each. Other repeat unit lengths are also known (e.g. 30, 33, 35, 39, 40, 42 amino acids). The last repeat in the repeat domain is usually only a half repeat of 19 or 20 amino acids length. The individual repeat units are generally not identical. They vary at certain variable amino acid positions, among these positions 12 and 13 are hypervariable while positions 4, 11, 24, and 32 vary with high frequency but at a lower frequency than 12 and 13 (variations at other positions occur also, but at lower frequency). The comparison of different AvrBs3-like proteins from *Xanthomonas* reveals 80 to 97 % overall sequence identity with most differences confined to the repeat domain. For example, AvrBs3 and the AvrBs3-like family member AvrBs4 differ exclusively in their repeat domain region, with the exception of a four amino acid deletion in the C-terminus of AvrBs4 with respect to AvrBs3.

A TALE polypeptide might be identified, for example, by the following method:
Synthesizing a polypeptide comprising a repeat domain, wherein the repeat domain comprises at least one repeat unit derived from a transcription activator-like (TAL) effector, wherein the repeat unit comprises a hypervariable region which determines recognition of a base pair in the target DNA sequence, wherein the repeat unit is responsible for the recognition of one base pair in the DNA sequence, and wherein the hypervariable region comprises a member selected from the group consisting of:
   (a) HD for recognition of C/G;
   (b) Nl for recognition of A/T;
   (c) NG for recognition of T/A;
   (d) NS for recognition of C/G or A/T or T/A or G/C;
   (e) NN for recognition of G/C or A/T;
   (f) IG for recognition of T/A;
   (g) N for recognition of C/G;
   (h) HG for recognition of C/G or T/A;
   (i) H for recognition of T/A; and
   (j) NK for recognition of G/C.

As outlined above, TALEs consist of a central domain of tandem repeats, nuclear localization signals (NLS) and an acidic transcriptional activation domain. The central repeat domain consists of almost identical tandem arranged 33-34 amino acid motifs that collectively mediate DNA binding.

The term "repeat domain" is used to describe the DNA recognition domain from a TALE polypeptide, or artificial version thereof that is made using the methods disclosed in the literature, consisting of modular repeat units that when present in a polypeptide confer target DNA specificity. A repeat domain comprised of repeat units can be added to any polypeptide in which DNA sequence targeting is desired and are not limited to use in TALEs.

The term "repeat unit" is used to describe the modular portion of a repeat domain from a TALE, or an artificial version thereof, that contains one amino acid or two adjacent amino acids that determine recognition of a base pair in a target DNA sequence. Repeat units taken together recognize a defined target DNA sequence and constitute a repeat domain. Repeat units can be added to any polypeptide in which DNA sequence targeting is desired and are not limited to use in TAL effectors.

A repeat domain preferably contains about 1.5 to 40.5 repeat units.

The term "functional part" of a TALE polypeptide as used herein means a fraction of the original polypeptide which still fulfils the function of a TALE polypeptide, i.e. the transcriptional activation of a gene, and at least contains one repeat unit of the central repeat domain of such a TALE polypeptide.

An "epigenetic inhibitor" as used herein means any substance which is able to resolve said heterochromatin structure around a gene thus making it accessible to the transcriptional machinery and for the TALE polypeptides.

In a preferred embodiment, the epigenetic inhibitor substance comprises at least one substance from the group of histone deacetylase (HDAC) inhibitors and/or DNA-methylation inhibitors. Preferred examples of these inhibitors are those, wherein the HDAC inhibitor is selected from the structural groups of small carboxylates, hydroxamic acids, benzamides, epoxyketones, and/or cyclic tetrapeptides.

More precisely, the HDAC inhibitor is preferably selected from small carboxylates and comprises valproic acid and/or butyrate. It is noted that the use of valproic acid is most preferred.

The HDAC inhibitor might further be selected from hydroxamic acids and comprise m-carboxy cinnamic acid bishydroxamic acid (CBHA), oxamflatin, PDX 101, pyroxamide, scriptaid, suberoylanilide hydroxamic acid (SAHA), LBH589 and/or NVP-LAQ824.

If the HDAC inhibitor is selected from cyclic tetrapeptides, then it may preferably comprise apicidin, depsipeptide, TPX-HA analogue, and/or trapoxin.

If the HDAC inhibitor in turn is selected from benzamides, it preferably comprises Cl-994 (N-acetyl dinaline) and/or MS-275.

Furthermore, as outlined above, a DNA methylation inhibitor might be used as an epigenetic inhibitor. Such a DNA methylation inhibitor preferably is selected from nucleoside analogues, preferably 5-azacytidine, 5-aza-2'-deoxycytidine, 5-fluoro-2'-deoxycytidine, 5,6-dihydro-5-azacytidine, and/or zebularine; and/or non-nucleoside analogues.

It should be noted that the above lists are illustrative only and show preferred embodiments. However, the skilled person will be able to define further substances useful in the present invention by routine experiments.

In a preferred embodiment, the present method involves the activation of differentiated cells. This is, for example outlined in the enclosed examples, where embryonic stem cells (ESCs) were differentiated into neural stem cells (NSCs). It was shown that in these differentiated cells the silenced endogenous *oct4* promoter could effectively be activated after treatment with TALEs and an epigenetic inhibitor.

As used herein, "endogenous" refers to nucleic acid or protein sequence naturally associated with a target gene or a host cell into which it is introduced.

In a further preferred embodiment, the method of the present invention comprises the further step of isolating cells with activated expression of a target gene subsequent to the treatment with a transcription activator like effector (TALE) polypeptide or a functional part thereof, and an epigenetic inhibitor substance.

The treatment might involve the concurrent or time staggered application of the transcription activator like effector (TALE) polypeptide or a functional part thereof, and the epigenetic inhibitor substance, to the cell.

As a preferred example, the treated cell might be a differentiated stem cell and the isolated cell might be a pluripotent stem cell.

In a second aspect, the present invention is directed to an isolated cell, preferably a pluripotent stem cell obtainable by the method as disclosed hereinabove. The cell may be preferably a plant cell, a human cell, animal cell, fungal cell or any other living cell. More preferably, the cell may be a pluripotent cell such as an embryonic stem cell (ESC).

An "isolated" cell is substantially or essentially free of components that normally accompany or interact with the cell as found in its naturally occurring environment.

In a further aspect, the present invention pertains to the use of at least one epigenetic inhibitor substance in combination with at least one transcription activator like effector (TALE) polypeptide or a functional part thereof, for activating expression of a target gene in a cell.

A still further aspect of the present invention provides a composition or kit-of-parts comprising
a) a TALE polypeptide or a functional part thereof, and
b) an epigenetic inhibitor substance.

The above defintions regarding both groups of substances also apply here.

The invention further relates to the field of plant and agricultural technology. In one aspect, the present invention is directed to a method to activate the expression of a target gene in plant cells, which method comprises providing plant cells with a TALE polypeptide according to the invention, said polypeptide being capable of specifically recognizing a target nucleotide sequence, or a complementary strand thereof, within a target gene, and allowing said polypeptide to recognize and particularly bind to said target nucleotide sequence, along with the treatment with an epigenetic inhibitor, whereby the expression of said target gene in said plant cells is activated.

The present invention is also directed to a method of treating a mammal, for example a human, suffering from a disease or detrimental physiological condition comprising the steps of obtaining tissues or cells from said mammal, modifying the tissues or cells with a composition or kit-of-parts as outlined above, and re-implanting the modified tissue/cells into the patient, in order to heal or at least ameliorate the disease or detrimental physiological condition.

Possible applications include the targeted reactivation of epigenetically silenced genes like e.g. tumor suppressor genes like e.g. the p16 tumor suppressor gene or the reactivation of the p53 tumor suppressor gene or the hyperactivation of mutated tumor suppressor genes (the rational here is that mutations reduce tumor suppressor function, and hyper-expression may compensate).

Alternatively, epigenetically silenced genes like, for example, the MECP2 gene could be reactivated in the therapy of the RETT syndrome.

Finally, regulatory genes could be activated to program cells. Fibroblast or other easily available cells are isolated from patients and *ex vivo* programmed into desired cell types for use in regenerative medicine. This is often referred to as reprogramming or transdifferentiation and has applications in cell based therapies of coronary heart diseases (after e.g. myocardial infarction) or neurodegenerative diseases or diabetes mellitus.

The following examples are offered by way of illustration and not by way of limitation.

### EXAMPLES

Specific control of gene activity is a valuable tool to study and engineer cellular functions. The inventors assembled plant pathogen derived DNA binding modules to generate designer transcription activator-like effector (dTALE) molecules. The inventors compared five distinct dTALEs binding at different sites within the *oct4* pluripotency gene promoter. Transcriptional activation greatly varied with their respective binding site and the epigenetic state of the target promoter and was greatly enhanced by HDAC inhibition.

The inventors generated five distinct dTALEs targeting the promoter of the murine pluripotency gene *oct4* to systematically test whether the position of the target sequence influences the efficiency of transcriptional activation. The inventors selected five 19-bp target sequences in the proximal *oct4* promoter, three upstream and two downstream of the Sp1/Sp3/hormone responsive element (HRE) box (**Fig. 1A**). DNA binding TALE repeat arrays were generated by multi-fragment cut-ligation using golden gate cloning and transferred to mammalian expression vectors by LR recombination (**Fig. 3**). To monitor transfection efficiency and expression levels, mcherry was fused to the N-terminus of the dTALEs. Furthermore, to compare the transcriptional activation of the wildtype and the VP16 activation domain (AD) each of the dTALEs was fused to both domains.

The activity of the different dTALEs was first analyzed in a transient reporter gene assay. HEK293T cells were co-transfected with an *oct4* promoter driven eGFP reporter gene (p*oct4-*GFP) and a constitutively expressed respective *dTALE* construct. Forty-eight hours after transfection the cells were harvested and the expression of the fluorescent proteins was analyzed by fluorescence measurement. All constructs were equally well expressed and exhibited nuclear localization (**Fig 4A**). Notably, the inventors did not exchange the TALE wildtype NLS that evolved to function in plants for mammalian homologues, demonstrating the high degree of conservation of this signal. Next, the inventors analyzed the level of transcriptional activation of the *oct4* promoter by the different *dTALE* constructs. All constructs induced the eGFP expression significantly, but with substantial variations among the different target sequences ranging from 4 to 25 fold (**Fig. 1C**). Interestingly, for both types of ADs the most distal dTALE (T-83) yielded the strongest transcriptional activation (**Fig. 1B** and **C**). The variation of the activity may be due to the relative distance to the transcriptional start site or to competition with endogenous factors, e.g. Sp1/Sp3.

Intriguingly, the *Xanthomonas* activation domain that evolved to activate plant host genes is also functional in mammalian cells. However, the *dTALE* genes encoding VP16 AD fusions (*VP16dTALEs*) induced significantly stronger expression of the p*oct4*-GFP construct than fusions with the wildtype activation domain *(wtdTALEs*; **Fig. 1C**). These different efficiencies were not caused by different expression levels as the mcherry signals showed that the wtdTALEs were even expressed at slightly higher levels than the VP16dTALEs (**Fig. 4A** and **B**). Taken together, these results demonstrate the importance of constructing and testing several dTALEs for a given target promoter to obtain the most effective activator.

In addition to positional effects, the inventors wanted to test whether the epigenetic state of the promoter might influence the efficiency of transcriptional activation. Therefore, the inventors methylated the p*oct4*-GFP plasmid *in vitro* using Sssl DNA methyltransferase. Digestion with the methylation sensitive restriction enzyme *Hpa*II showed complete protection of the *in vitro* methylated plasmid (**Fig. 5A**). The inventors then tested this modified plasmid DNA in transient *oct4* reporter gene assays as described above. All dTALE constructs successfully induced expression of eGFP from the methylated *oct4* promoter (**Fig. 1D**). Interestingly, most of the dTALEs achieved relative eGFP expression levels comparable to those of the unmethylated promoter, the only exception being the T-83 construct which activated the transcription up to 35 and 65 fold with wildtype AD and VP16AD fusions, respectively (**Fig. 1D**). The strong increase of relative expression is possibly due to the greatly diminished basal transcription of methylated reporter constructs, while the absolute eGFP fluorescence was reduced (**Fig. 5B** and **5C**). The observed differences in transcription activation efficiency were more pronounced with methylated templates probably reflecting local epigenetic variations in form of nucleosome positioning and/or repressive marks.

To test the ability of dTALEs to activate the endogenous *oct4* gene the inventors generated mouse embryonic stem cells (ESCs) stably carrying the p*oct4*-GFP reporter construct. The p*oct4*-GFP ESCs (ogESCs) were tested with the most efficient T-83 dTALE fused to the VP16 AD in comparison with mcherry control vectors. Transfected mcherry positive cells were isolated using fluorescent activated cell sorting (FACS) and total RNA was isolated followed by reverse transcription and quantitative real-time PCR (RT-PCR). FACS analysis showed that ogESCs transfected with the *dTALE* construct had a three to fourfold higher mean eGFP fluorescence intensity compared to control transfected cells (**Fig. 2A**) and transcription of endogenous *oct4* was induced about threefold as determined by RT-PCR (**Fig. 2B**). These results demonstrate that it is possible to hyperactivate the endogenous *oct4* locus with dTALEs and that there is a good correlation between activation of transgenic and endogenous sequences. The relatively low induction rate in this assay is likely due to the high basal level of *oct4* transcription in ESCs and the negative feedback of Oct4 on its own promoter.

To test whether dTALEs can also activate a silent endogenous *oct4* promoter, the inventors differentiated ogESCs into neural stem cells (NSCs). During this process the *oct4* locus becomes silenced and NSCs no longer express *oct4*. The OGESC-derived NSCs (ogNSCs) were transfected with the T-83 construct or vector expressing only mcherry. 72 hours after transfection cells were harvested and analyzed by flow cytometry. In this assay, no increase in eGFP positive cells was observable (**Fig. 2C**). This may be due to the robustness of epigenetic repressive mechanisms acting on the *oct4* promoter preventing uncontrolled activation. The inventors therefore treated the ogNSCs with two different histone deacetylase inhibitors, namely Trichostatin A (TSA)⁹ and Valproic acid (VPA)¹⁰ as well as with 5-azadeoxycytidine (5-aza-dC) a commonly used mechanism-based DNA methyltransferase inhibitor¹¹. To find the optimal concentrations of the inhibitors cells were transfected with the *dTALE* and plated in medium containing dilutions of the respective inhibitors. Forty-eight hours after transfection cells were analyzed by flow cytometry. While TSA treatment did not significantly increase reporter gene expression (**Fig. 2D**), VPA treatment efficiently enhanced induction of eGFP expression by the dTALE up to 25 fold (**Fig. 2E**). Similarly, transfected cells cultured in the presence of 5-aza-dC showed strong induction of the oct4 reporter expression compared to untreated cells (**Fig. 2F**). To analyze if epigenetic inhibitors could also enhance the activation of the endogenous *oct4* gene by dTALEs, cells were sorted and oct4 mRNA levels were analyzed. While transfected ogNSCs treated with 3 nM TSA failed to induce expression of the endogenous *oct4,* cells treated with 620 µM VPA or 10nM 5-AzadC, respectively, showed a greatly enhanced *oct4* expression (**Fig. 2G**).

This demonstrates that it is possible to overcome the epigenetic barrier that prevents the transcriptional activation of the *oct4* promoter by dTALEs using epigenetic inhibitors and may have strong implications for the generation of induced pluripotent stem cells, as activation of the endogenous *oct4* is a key event in the process of cellular reprogramming.

In summary, these results show that dTALEs can be designed to bind any genomic sequence and activate individual mammalian genes. Furthermore, the inventors demonstrate that the epigenetic state of the promoter is a crucial aspect in dTALE mediated gene activation and that the efficiency of transcriptional activation of silenced/methylated genes can be greatly enhanced by treatment with epigenetic inhibitors.

### MATERIALS AND METHODS

### Construction of dTALEs

A Gateway cassette from pGWB5 was amplified (forward primer: 5'-GGGGCGATCGCACAAGTTTGTACAAAAAAGCTGAACGAG-3'; **reverse primer**: 5'-GGGGCGGCCGCAACCACTTTGTACAAGAAAGCTGAACG-3'), thereby adding AsiSl and *Not*I restriction sites. This fragment was cloned via *AsiS*I and *Not*I into pCAG_mCh¹⁶ generating pCAG_mCh_GW. The VP16AD was amplified from RSV E2F1-VP16¹⁷ (forward primer: 5'-GGGGGTCTCTCACCATGGATCCTGCCCCCCCGACCGATGTCAGC-3'; reverse primer: 5'-GGGGGTCTCCCTTCTACCCACCGTACTCGTCAATTCCAAGG-3'), thereby adding a *Bam*HI restriction site to the 5' end and cloned into pENTR-D-TOPO (Invitrogen) generating pENTR-D-*Bam*HI_VP16AD. TALE repeat arrays were generated via multi-fragment cut-ligation using golden gate cloning and ligated either into pENTR-D-TALE-Δrep-*Bpi*I-AC or pENTR-D-TALE-Δrep-*Bpi*I-AC-VP16AD. All entry clones were transferred by LR recombination (Invitrogen) into the expression vector pCAG_mCh_GW.

### Plasmid construction

The *oct4* reporter construct (p*oct4*-GFP) was generated by inserting the X*ho*I*lAvr*II fragment of **GOF**-18 together with a linker oligo (5'-CCTAGGTGAGCCGTCTTTCCAC CAGGCCCCCGGCTCGGGGTGCGATCGCCGCCCATGG-3') into pGL-3 basic (Promega) cut with *Xho*I/*Nco*I. Subsequently, the Luciferase ORF was removed by cutting with *Kas*I/*Fse*I and the eGFP ORF (amplified with: forward primer: 5'-AAAGGCGCCAGTGAGCAAGGGCG-3'; reverse primer: 5'-AAAGGCCGGCCTTACTTGTAC AGCTCGTCC-3') was inserted.

### Cell culture, transfection and FACS sorting

HEK293T cells cultured in DMEM supplemented with 50 µg/ml gentamicin and 10% FBS. For expression of fusion proteins HEK293T cells were transfected with polyethylenimine (Sigma). Mouse ESCs were cultured as described. Mouse NCSs were cultured in N2B27 supplemented with 20ng/ml FGF2 and EGF. NSCs and ESCs were transfected using Lipofectamin 2000 (Invitrogen) according to the manufacturer's instructions. ESCs and NSCs were sorted with a FACS Aria II instrument (Becton Dickinson).

### Generation of transgenic cell lines

p*oct4*-GFP transgenic ESCs (ogESCs) were generated by transfecting cells with the p*oct4-*GFP reporter construct and sorting for eGFP expression 48 hours after transfection. Subsequently, a pool of ESCs stably carrying the *oct4* reporter construct was obtained by three rounds of culture and repeated FACS sorting of eGFP expressing cells. Finally, single cell sorting was used to obtain a clonal transgenic cell line.

### In vitro generation of neural stem cells (NSCs)

ogESCs were differentiated to ogNSCs as previously described. In brief, 3,5x10⁵ cells were plated in a 25cm² culture flask with N2B27 medium containing 1000 U/ml LIF (ESGRO, Millipore). The next day the medium was exchanged against N2B27 without LIF to initiate differentiation into the neural lineage. After 7 days cells were plated in Euromed-N (Euroclone) supplemented with 20 ng/ml EGF and FGF2 (Peprotech). After 5 days neurospheres were collected and plated in N2B27 medium containing 20 ng/ml EGF and FGF2 to allow outgrowth of NSCs.

### In vitro methylation & reporter gene assay

*In vitro* methylation of p*oct4*-GFP was conducted using M.Sssl (New England Biolabs). 45 U of enzyme were incubated with 45 µg of plasmid DNA in the presence of 160 µM SAM over night. After 3 hours of incubation another 160 µM of SAM was added to ensure complete methylation. Methylation status of the plasmid after *in vitro* methylation was tested by digestion with *Msp*I and *Hpa*II (Fermentas). For the reporter gene assay HEK293T cells were plated in a p35 and grown to 70% confluence. Subsequently, cells were co-transfected with the poct4-GFP reporter plasmid and the respective TAL effector construct. 48 h after transfection cells were lysed in PBS containing 0,5% NP40 and mammalian protease inhibitors. The lysate was cleared and fluorescence intensities of eGFP and mcherry were measured with a Tecan infinite M1000 plate reader.

### RNA Isolation, cDNA synthesis and quantitative Real-time PCR

Isolation of RNA and reverse transcription was carried out as described previously. Real Time PCR analysis was performed on the 7500 Fast Real-Time PCR System (Applied Biosystems) at standard reaction conditions using TaqMan Gene Expression Master Mix (Applied Biosystems) and the following TaqMan Gene expression assays: gapdh (Assay ID: Mm99999915_g1) and oct4 (Assay ID: Mm00658129_gH). Relative mRNA levels were normalized to gapdh and calculated with the comparative C_{T} Method (ΔΔC_{T} Method).

### References

1. Zhang, F. et al. Efficient construction of sequence-specific TAL effectors for modulating mammalian transcription. Nat Biotech 29, 149-153 (2011).
2. Miller, J.C. et al. A TALE nuclease architecture for efficient genome editing. Nat Biotech 29, 143-148 (2010).
3. Morbitzer, R., Römer, P., Boch, J. & Lahaye, T. Regulation of selected genome loci using de novo-engineered transcription activator-like effector (TALE)-type transcription factors. Proc Natl Acad Sci U S A (2010) 107, 21617-21622 (2010)
4. Mahfouz, M.M. et al. De novo-engineered transcription activator-like effector (TALE) hybrid nuclease with novel DNA binding specificity creates double-strand breaks. Proc Natl Acad Sci USA, 108, 2623-2628 (2011)
5. Bogdanove, A.J., Schornack, S. & Lahaye, T. TAL effectors: finding plant genes for disease and defense. Curr. Opin. Plant Biol 13, 394-401 (2010).
6. Römer, P. et al. Plant pathogen recognition mediated by promoter activation of the pepper Bs3 resistance gene. Science 318, 645-648 (2007).
7. Kay, S., Hahn, S., Marois, E., Hause, G. & Bonas, U. A bacterial effector acts as a plant transcription factor and induces a cell size regulator. Science 318, 648-651 (2007).
8. Boch, J. et al. Breaking the code of DNA binding specificity of TAL-type III effectors. Science 326, 1509-1512 (2009).
9. Yoshida, M., Kijima, M., Akita, M. & Beppu, T. Potent and specific inhibition of mammalian histone deacetylase both in vivo and in vitro by trichostatin A. J. Biol. Chem 265, 17174-17179 (1990).
10. Göttlicher, M. et al. Valproic acid defines a novel class of HDAC inhibitors inducing differentiation of transformed cells. EMBO J 20, 6969-6978 (2001).
11. Jones, P.A. & Taylor, S.M. Cellular differentiation, cytidine analogs and DNA methylation. Cell 20, 85-93 (1980).

## Claims

1. A method of activating expression of a target gene in a cell, wherein the cell is treated with at least one transcription activator like effector (TALE) polypeptide or a functional part thereof,
**characterized in that**
the cell is further treated with at least one epigenetic inhibitor substance.

2. The method of claim 1, wherein the target gene comprises a transcriptional control element for a gene, preferably a promoter or an enhancer.

3. The method of claim 1 or 2, wherein the target gene comprises a transcriptionally or epigenetically silenced promoter or enhancer.

4. The method of claim 3, wherein the promoter is a pluripotency gene promoter, preferably the *oct4* pluripotency gene promoter.

5. The method of one or more of the preceding claims, wherein the functional part of the TALE polypeptide at least contains one repeat unit of the central repeat domain of a TALE polypeptide.

6. The method of one or more of the preceding claims, wherein the epigenetic inhibitor substance comprises at least one substance from the group of histone deacetylase (HDAC) inhibitors and/or DNA-methylation inhibitors.

7. The method of claim 6, wherein the HDAC inhibitor is selected from the structural groups of small carboxylates, hydroxamic acids, benzamides, epoxyketones, and/or cyclic tetrapeptides.

8. The method of claim 7, wherein the HDAC inhibitor is selected from small carboxylates and comprises valproic acid and/or butyrate; and/or
wherein the HDAC inhibitor is selected from hydroxamic acids and comprises m-carboxy cinnamic acid bishydroxamic acid (CBHA), oxamflatin, PDX 101, pyroxamide, scriptaid, suberoylanilide hydroxamic acid (SAHA), LBH589 and/or NVP-LAQ824; and/or
wherein the HDAC inhibitor is selected from cyclic tetrapeptides and comprises apicidin, depsipeptide, TPX-HA analogue, and/or trapoxin; and/or
wherein the HDAC inhibitor is selected from benzamides and comprises Cl-994 (N-acetyl dinaline and/or MS-275.

9. The method of one or more of the preceding claims, wherein the DNA methylation inhibitor is selected from nucleoside analogues, preferably 5-azacytidine, 5-aza-2'-deoxycytidine, 5-fluoro-2'-deoxycytidine, 5,6-dihydro-5-azacytidine, and/or zebularine; and/or non-nucleoside analogues.

10. The method of one or more of the preceding claims, which involves the activation of differentiated cells.

11. The method of one or more of the preceding claims, comprising the further step of isolating cells with activated expression of a target gene subsequent to the treatment with a transcription activator like effector (TALE) polypeptide or a functional part thereof, and an epigenetic inhibitor substance.

12. The method of claim 11, wherein the treatment involves the concurrent or time staggered application of the transcription activator like effector (TALE) polypeptide or a functional part thereof, and the epigenetic inhibitor substance, to the cell, wherein the treated cell preferably is a differentiated stem cell and the isolated cell preferably is a pluripotent stem cell.

13. An isolated cell, preferably a pluripotent stem cell obtainable by the method of one or more of claims 11-12.

14. Use of at least one epigenetic inhibitor substance in combination with at least one transcription activator like effector (TALE) polypeptide or a functional part thereof, for activating expression of a target gene in a cell.

15. A composition or kit-of-parts comprising
a) a transcription activator like effector (TALE) polypeptide or a functional part thereof, and
b) an epigenetic inhibitor substance.
